# EUROPEAN PATENT APPLICATION

(11) **EP 1 712 219 A1**
(43) Date of publication of application: **18.10.2006**
(21) Application number: 05709665.3
(22) Date of filing: 03.02.2005
(51) Int. Cl.: A61K 8/18

(54) **WRINKLE-DIMINISHING AGENT**

(30) Priority: 04.02.2004 JP 2004027613; 07.06.2004 JP 2004197414
(71) Applicant: Kao Corporation, Chuo-ku Tokyo 103-8210 (JP)
(72) Inventor: MIURA, Kyoko, (JP); HARATAKE, Akinori, (JP); SAKAI, Shingo, (JP)
(74) Representative: Murphy, Colm Damien
(86) International application number: PCT/JP2005/001572
(87) International publication number: WO 2005/074879

(57) **Abstract**

An antiwrinkle agent which is highly effective in alleviating wrinkles caused by the photoaging. The present invention relates to an antiwrinkle agent comprising an N-acetylglucosamine organic acid ester represented by the following general formula (1): 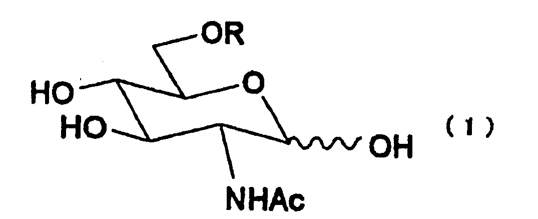 wherein R is a linear or branched acyl group having 2 to 18 carbon atoms and the configuration in the 1-position may be either α-form or β-form.

## Description

### Field of the Invention

The present invention relates to an antiwrinkle agent comprising an N-acetylglucosamine organic acid ester having excellent wrinkle alleviating effect on the wrinkles formed due to aging, especially on exposed skin portions, and being capable of keeping skins healthy from the viewpoint of dermatology and beauty.

### Background Art

In all living bodies including human being, organs gradually deteriorate after they are born and as they grow old, and the functions of some of them stop later and the number of the organs of which functions have stopped reaches a certain value or more, resulting in a death. A process in which the functions gradually deteriorate is called aging. Skins are affected directly by their environments, and have an important function to maintain the conditions inside a living body and hence all functions of the skins rarely stop, but the skin is an organ likely to remarkably show signs of aging, such as wrinkles or spots, being of dull color, or being loose, which are marked especially at skin portions exposed to daylight.

As the skin ages, protection of the skin against stimulation, such as oxidation stress, weakens to cause the conditions inside the skin to be bad, thus promoting the aging. Particularly, the skin portion exposed to daylight is always exposed to strong oxidation stress such as ultraviolet radiation, and hence the progress of aging is remarkable in the exposed skin. Such a change of the skin is called photoaging. In the skin which has suffered from photoaging, various changes such as decrease in collagen which is a major constituent of the dermis occur to cause deep or large wrinkles on the skin surface, leading to beauty problems.

As a substance having a wrinkle alleviating effect on the wrinkles caused by the photoaging, retinoic acid is used as a prescription drug in the United States, but the retinoic acid has a strong side effect and has a problem from the viewpoint of safety, and hence it has not yet been approved in Japan (See, "FRAGRANCE JOURNAL", published on April 15, 1998, Vol.26, No.4, p. 75-77). Therefore, a development of a satisfactorily effective and highly safe substance for alleviating the wrinkles is desired.

### DISCLOSURE OF THE INVENTION

### Problems to be Solved by the Invention

It is an object of the present invention to provide an antiwrinkle agent having excellent wrinkle alleviating effect on the wrinkles markedly formed due to aging, especially on exposed skin portions, and being capable of keeping skins healthy from the viewpoint of beauty.

In view of the above, the present inventors have conducted extensive and intensive studies. As a result, it has been found that the antiwrinkle agent shown below is advantageous not only in that it has excellent wrinkle alleviating effect on the wrinkles markedly formed due to aging, especially on exposed skin portions, but also in that it can keep skins healthy from the viewpoint of beauty while securing excellent safety, and thus the present invention has been completed.

The present invention is directed to an antiwrinkle agent comprising an N-acetylglucosamine organic acid ester represented by the following general formula (1): 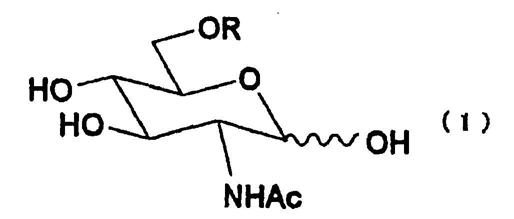 wherein R is a linear or branched acyl group having 2 to 18 carbon atoms and the configuration in the 1-position may be either α-form or β-form.

In the present invention, there is provided an antiwrinkle agent which is advantageous not only in that it has excellent wrinkle alleviating effect on the wrinkles formed due to aging, especially on exposed skin portions, but also in that it keeps skins healthy from the viewpoint of dermatology and beauty.

### BEST MODE FOR CARRYING OUT THE INVENTION

Hereinbelow, embodiments of the present invention will be described in detail.

N-Acetylglucosamine is a kind of amino sugars, which are contained in a large amount in the chitin material constituting the outer shells of crustaceans, such as crabs and lobsters. The N-acetylglucosamine organic acid ester used in the present invention can be obtained by esterifying an N-acetylglucosamine using an organic acid by a general method.

The N-acetylglucosamine organic acid ester used in the present invention is represented by the following general formula (1) : 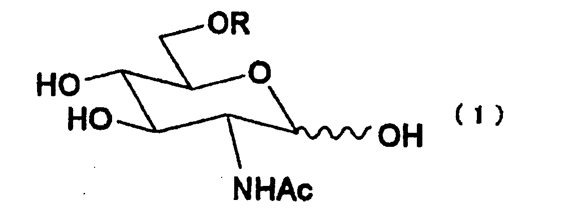 wherein R is a linear or branched acyl group having 2 to 18 carbon atoms and the configuration in the 1-position may be either α-form or β-form.

In the general formula (1), R preferably has 2 to 12 carbon atoms, more preferably 8 to 12 carbon atoms. R may be either saturated or unsaturated. Examples of R include acetyl, butanoyl, heptanoyl, hexanoyl, octanoyl, 2-ethylhexanoyl, lauroyl, palmitoyl, stearoyl and oleoyl.

In the present invention, the configuration in the 1-position may be either α-form or β-form, and any one of them or a mixture thereof can be used.

Specific examples of the general formula (1) include the following general formula (2):

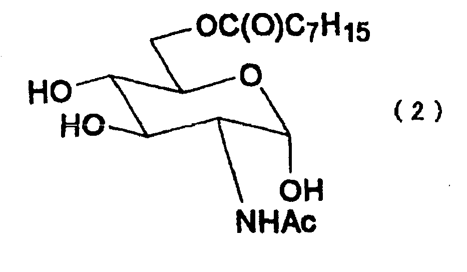

In the present invention, it is preferred that the amount of the N-acetylglucosamine organic acid ester formulated is 0.001 to 10.0% by weight (hereinafter, "%" is given by weight unless otherwise specified), based on the total weight of the antiwrinkle agent. When the amount is in this range, the effect aimed at by the present invention can be efficiently and sufficiently achieved. The amount is more preferably 0.05 to 5.0%, further preferably 0.1 to 2.0%.

The antiwrinkle agent of the present invention can be formulated in cosmetics for skin, external drug preparation or bath salts, and can be used in the form of, for example, lotion, emulsion, cream or pack.

In the antiwrinkle agent of the present invention, in addition to the above-described component, a coloring agent, a fragrance, a preservative, a surfactant, a pigment or an antioxidant can be added in such an amount that the effect of the present invention is achieved.

### Examples

The present invention will be described in more detail with reference to the following Examples and Comparative Examples, which should not be construed as limiting the scope of the present invention.

### Preparation Example 1

### Preparation method for 2-acetamide-2-deoxy-6-O-octanoyl-α-D-glucopyranose (compound 1)

5 mL of pyridine and 5 mL of N,N-dimethylformamide were added to 0.5 g of N-acetylglucosamine and heated to 70°C while stirring, and 0.46 mL of n-octanoyl chloride was added dropwise to the resultant mixture to effect a reaction for four hours. After completion of the reaction, the resultant reaction mixture was extracted with ethyl acetate and washed with 2 mol/L hydrochloric acid, and then the ethyl acetate layer was dried by anhydrous magnesium sulfate, followed by removal of the solvent under a reduced pressure. The resultant residue was purified by silica gel column chromatography (elution solvent: chloroform:methanol = 15:1) to obtain 170 mg of 2-acetamide-2-deoxy-6-O-octanoyl-α-D-glucopyranose in the form of white crystal.

The results of 1H-NMR measurement of 2-acetamide-2-deoxy-6-O-octanoyl-α-D-glucopyranose are shown below.

NMR (DMSO-d6) δ: 0.92 (t, 3H, J = 6.8 Hz), 1.33 (s, 10H), 1.55-1.60 (m, 2H), 1.89 (s, 3H), 2.34 (t, 2H), 3.15-3.20 (m, 1H), 3.55-3.60 (m, 1H), 3.65-3.70 (m, 1H), 3.85-3.90 (m, 1H), 4.08 (dd, 1H, J = 6.0, 11.6 Hz), 4.35 (dd, 1H, J = 2.1, 11.8 Hz), 4.70 (d, 1H, J = 5.4 Hz), 4.96 (t, 1H, J = 3.5, 4.3 Hz), 5.13 (d, 1H, J = 5.8 Hz), 6.54 (d, 1H, J = 4.7 H), 7.61 (d, 1H, J = 8.1 Hz).

### Example 1 and Comparative Example 1

The wrinkle alleviating effect of sample comprising only base or of sample comprising compound 1, when applied to a skin which had suffered from photoaging, was examined by the following test method.

### 1. Experimental animal

A group of ten hairless mice which were ten weeks old at the start of the test was used.

### 2. Evaluation of wrinkle alleviating effect

### 2-1. Photoaging conditions and evaluation method

Photoaging was caused by irradiating the mice with UVA and UVB once a day and five times per week for continuous eight weeks. A UVA dose of 20 J/cm² was increased to 25 J/cm² and then 30 J/cm² every week, and a UVB dose of 20 mJ/cm² was increased to 30 mJ/cm² and then 40 mJ/cm² every week, and the respective maximum doses were used on and after the third week.

The wrinkle alleviating effect was evaluated in respect of the wrinkle score and the dermis collagen content. Wrinkle score is rated in accordance with the method of Bissett et. al. (Photochem Photobiol 46:367-378, 1987). Specifically, the wrinkles were macroscopically evaluated with respect to the size and depth and rated with maximum of point 3 as follows: "large and deep wrinkles can be recognized" was rated 3, "some wrinkles can be recognized" was rated 2, "any wrinkles cannot be recognized" was rated 1, and "normal and fine skin is observed" was rated 0.

Dermis collagen content is determined as follows:whole skin was sampled and homogenized by a POLYTRON Homogenizer (manufactured by KINEMATICA AG), and then the collagen fraction was extracted and subjected to acid hydrolysis followed by quantitative determination of a hydroxyproline content as for collagen content using an amino acid analyzer (manufactured by JASCO Corporation).

### 2-2. Sample and experimental procedure

N-acetylglucosamine organic acid ester (compound 1) was added to a 50% (v/v) aqueous ethanol solution (base) in the concentrations of 1% (Example 1). In Comparative Example 1, only the base was used.

0.1 mL of each of these samples was applied to the hairless mice at their dorsal skins (diameter: about 2.5 cm) once a day and five times per week from the fifth week after the start of the UV irradiation to the fourth week after the termination of the irradiation. After the final application, wrinkle scores were obtained. After killing the mice, a skin was sampled. The collagen content was measured as hydroxyproline content per square centimeter. The wrinkle alleviating effect was evaluated by comparing the wrinkle score and the collagen content with those in which only the base was applied.

### 3. Evaluation

The results of wrinkle scores

| Group | Wrinkle score |
|---|---|
| Example 1 (sample comprising compound 1) applied | 2.20 ± 0.11 |
| Comparative Example 1 (sample comprising only base) applied | 2.55 ± 0.11 |
| (Value is average ± standard error.) | |

The wrinkle score in Example 1 is significantly low as compared to that in Comparative Example 1, which indicates that the antiwrinkle agent comprising the N-acetylglucosamine organic acid ester is effective against the wrinkles caused due to the photoaging.
The results of the measurements of the collagen content

| Group | Collagen content (µmol/cm²) |
|---|---|
| Example 1 (sample comprising compound 1) applied | 7.84 ± 0.40 |
| Comparative Example 1 (sample comprising only base) applied | 7.19 ± 0.73 |
| (Value is average ± standard error.) | |

The collagen content in Example 1 was significantly high as compared to that in Comparative Example 1, which indicates that the N-acetylglucosamine organic acid ester is effective against the reduction of the dermis collagen content due to the photoaging.

From the results of the present tests, it is apparent that the antiwrinkle agent (Example 1) comprising the N-acetylglucosamine organic acid ester (compound 1) prepared by Preparation Example 1 has a remarkable wrinkle alleviating effect on the wrinkles caused by the photoaging as compared to the Comparative Example 1.

### Example 2 and Comparative Example 2

Skin lotions having the compositions shown below were individually prepared by the method described below, and the lotions prepared as samples were evaluated with respect to the wrinkle alleviating effect in accordance with the following procedure.

Five healthy persons (women; age: 45 to 57) having wrinkles at the corners of their eyes were selected as subjects, and they applied each of the skin lotions in Example and Comparative Example. After the final application, a questionnaire survey about the conditions of the skins (wrinkles) at the corners of their eyes was conducted in accordance with the following procedure.

The subjects individually applied about 0.2 ml of each of the samples to the respective portions of wrinkles at the corners of the left eye and the right eye (about 4 cm² or 2 cm x 2 cm around the corner of the eye per sample) after washing the face in the morning and after bathing in the evening, i.e., twice a day for continuous two months (sixty days). After the final application, they filled in a questionnaire about the conditions of the skins (wrinkles) at the corners of their left and right eyes.

### 1. Composition of skin lotion:

| Ingredients | Amount (%) |
|---|---|
| Ingredient A | |
| Olive oil | 10.0 |
| Isopropyl myristate | 1.0 |
| Polyoxyethylene (20) sorbitan monolaurate | 0.5 |
| Propylene glycol | 1.0 |
| Glycerol | 2.0 |
| | |

| Ingredient B | |
|---|---|
| Methylparaben | 0.1 |
| Ethanol | 7.0 |
| Purified water | Balance |
| | |

| Ingredient C | |
|---|---|
| Compound 1 | 1.0 (Example 2) or 0 (Comparative Example 2) |

### 2. Preparation method

N-acetylglucosamine organic acid ester (compound 1) as ingredient C was added to ingredient B and uniformly dissolved, and then ingredient A was added and the resultant mixture was dispersed by stirring and then filled in a container. The contents of the container were uniformly dispersed by shaking before being used.

### 3. Evaluation

Based on the feedback of the questionnaire, the numbers of the persons who answered that the skin lotion in Example 3 was more effective than that in Comparative Example 2 in individual items for the conditions of the skins (wrinkles) are shown below.

| Item | Number of persons |
|---|---|
| Wrinkles alleviated. | 5 |
| Skin softened. | 5 |
| Skin tensed. | 5 |
| Skin lustered. | 3 |
| Skin brightened. | 3 |

From the results of the present test, it is apparent that the skin lotion in Example 2 considerably alleviated the wrinkles, as compared to that in Comparative Example 2, and further it improved the softness or tenseness of the skin which deteriorated due to the photoaging. In addition, the skin lotion in the present invention caused no skin troubles, e.g., no stimulation or no itching.

### Example 3

Skin cream having the compositions shown below was prepared by the method described below, and twenty healthy persons (women; age: 48 to 57), who preliminarily answered that they had skin troubles of wrinkles at the corners of their eyes, used the skin cream for one week or longer and then filled in a questionnaire.

### 1. Composition of skin cream

| Ingredients | Amount (%) |
|---|---|
| Ingredient A | |
| Bees wax | 2.0 |
| Stearic acid | 5.0 |
| Stearyl alcohol | 5.0 |
| Reduced lanolin | 2.0 |
| Squalene | 20.0 |
| Sorbitan monostearate | 3.0 |
| Polyoxyethylene (20) sorbitan monostearate | 3.0 |
| Propylene glycol | 5.0 |
| | |

| Ingredient B | |
|---|---|
| Methylparaben | 0.2 |
| Purified water | Balance |
| | |

| Ingredient C | |
|---|---|
| Compound 1 | 1.0 |

### 2. Preparation method

N-acetylglucosamine organic acid ester (compound 1) as ingredient C was added to ingredient B, and then ingredients A and B were individually dissolved by heating to 80°C and mixed, and cooled to 30°C while stirring to prepare a skin cream.

### 3. Evaluation

After the subjects individually used the skin cream in Example 3, they filled in a questionnaire about the state of wrinkles in the items shown below. The numbers of the persons who answered that the description in each item was true after using the skin cream are shown below.

| Item | Number of persons |
|---|---|
| Wrinkles alleviated. | 18 |
| Wrinkles reduced in size. | 18 |
| Wrinkles reduced in number. | 6 |
| Wrinkles increased. | 0 |

From the results of the present evaluation, it is apparent that almost all the subjects sensed that their wrinkles alleviated as compared to them before use in Example 3, and the fact that the skin cream was more effective in reducing the size of wrinkles rather than in reducing the number of wrinkles indicates that the skin cream alleviated the wrinkles caused by the photoaging. In addition, the skin cream in the present invention caused no skin troubles, e.g., no stimulation or no itching.

### INDUSTRIAL APPLICABILITY

The antiwrinkle agent of the present invention can be formulated in cosmetics for skin, external drug preparation or bath salts, and can be used in the form of, for example, lotion, emulsion, cream or pack, and it is very useful from the viewpoint of skin beauty.

## Claims

1. An antiwrinkle agent comprising an N-acetylglucosamine organic acid ester represented by the following general formula (1) : 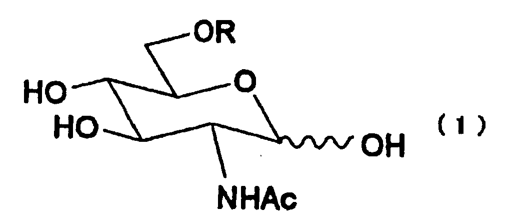 wherein R is a linear or branched acyl group having 2 to 18 carbon atoms and the configuration in the 1-position may be either α-form or β-form.

2. The antiwrinkle agent according to claim 1, wherein the N-acetylglucosamine organic acid ester is represented by the following general formula (2):

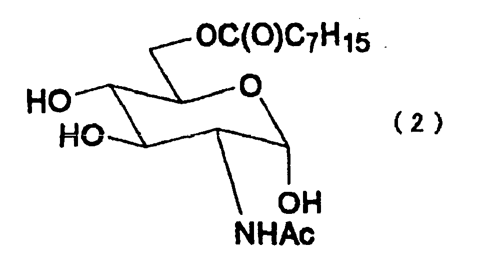

3. The antiwrinkle agent according to claim 1 or 2, wherein the amount of the N-acetylglucosamine organic acid ester formulated is 0.001 to 10.0% by weight, based on the weight of the antiwrinkle agent.
